# EUROPÄISCHE PATENTSCHRIFT

(11) **EP 4 464 296 B1**
(45) Veröffentlichungstag und Bekanntmachung des Hinweises auf die Patenterteilung: **18.11.2026**
(21) Anmeldenummer: 24164950.8
(22) Anmeldetag: 20.03.2024
(51) Int. Cl.: A61G 12/00

(54) **DECKENBEFESTIGUNGSVORRICHTUNG FÜR MEDIZINGERÄTE**
CEILING MOUNTING DEVICE FOR MEDICAL DEVICES
DISPOSITIF DE FIXATION AU PLAFOND POUR APPAREILS MÉDICAUX

(30) Priorität: 16.05.2023 DE 202023102647 U
(43) Veröffentlichungstag der Anmeldung: 20.11.2024
(73) Patentinhaber: Amtrion GmbH, 32457 Porta Westfalica (DE)
(72) Erfinder: ROSNER, Matthias, 32549 Bad Oeynhausen (DE)
(74) Vertreter: Wickord, Wiro

(56) Entgegenhaltungen:
- WO-A1-2020/197522
- CN-U- 207 864 975
- DE-A1- 10 323 169

## Beschreibung

Die Erfindung betrifft eine Deckenbefestigungsvorrichtung für Medizingeräte nach dem Oberbegriff des Patentanspruchs 1.

Deckenbefestigungsvorrichtungen werden beispielsweise in Operationssälen, Notaufnahmen oder anderen medizinischen Behandlungsräumen eingesetzt, um Medizingeräte deckenhängend und bevorzugt schwenkbar beziehungsweise höhenverstellbar zu installieren. Wird eine derartige Deckenbefestigungsvorrichtung neu installiert, wird anhand der Planungsunterlagen oder vor Ort ein Aufmaß genommen und abhängig von der gewünschten Position, die das Medizingerät später einnehmen soll, die Länge des Deckenrohrs ermittelt. Das Deckenrohr wird anschließend auftragsspezifisch nach Maß gefertigt und lackiert und dann zusammen mit den anderen Komponenten der Deckenbefestigungsvorrichtung kommissioniert. Sofern bei der Installation der Deckenbefestigungsvorrichtung später festgestellt wird, dass die Länge des beigestellten Deckenrohrs nicht der Spezifikation entspricht oder sich die Randbedingungen vor Ort beziehungsweise die Vorgaben für die Aufhängung des Medizingeräts zwischenzeitlich geändert haben, muss entweder ein neues Deckenrohr beschafft und die weitere Montage ausgesetzt werden oder das beigestellte Deckenrohr auf der Baustelle eingekürzt werden. Hierbei wird dann jedoch die werkseitig hergestellte Lackierung des Deckenrohrs beschädigt.

Eine gattungsgemäße Deckenbefestigungsvorrichtung für Medizingeräte ist aus der WO 2020/197522 A1 bekannt.

Aufgabe der vorliegenden Erfindung ist es, eine verbesserte Deckenbefestigungsvorrichtung und eine verbesserte Installationsanordnung hiermit anzugeben.

Zur Lösung der Aufgabe weist die Erfindung die Merkmale des Patentanspruchs 1 auf.

Der besondere Vorteil der Erfindung besteht darin, dass die Länge des Deckenrohrs der zu installierenden Deckenbefestigungsvorrichtung vor Ort auf der Baustelle variabel eingestellt beziehungsweise passend gewählt werden kann. Hierzu werden standardisierte, industriell gefertigte Deckenrohrsegmente unterschiedlicher Länge bedarfsgerecht zu einem Deckenrohr kombiniert. Die Länge des Deckenrohrs kann so ohne eine Unterbrechung der Installation und ohne eine Beschädigung der Lackierung an gegebenenfalls geänderte Randbedingungen angepasst werden. Die nach dem Baukastenprinzip in unterschiedlichen Längen hergestellte Rohrsegmente lassen sich so in gleichbleibender Qualität kostengünstig herstellen.

Um zu verhindern, dass die Rohrsegmente, die gemeinsam das Deckenrohr bilden, sich mit der Zeit lösen, sind benachbarte Rohrsegmente über ein Verschraubungssicherungselement gesichert. Das Verschraubungssicherungselement ist so ausgebildet, dass es bei einer bestimmungsgemäßen Verwendung der Deckenbefestigungsvorrichtung einem unbeabsichtigten Lösen der Verschraubung entgegenwirkt. Zugleich kann vorgesehen sein, dass sich die Verschraubung bei einer Demontage beispielsweise durch ein aktives Lösen der Verschraubungssicherung oder das Aufbringen einer erhöhten Demontagekraft lösen lässt.

Als Verschraubungssicherungselement kann beispielsweise eine Zugkordel vorgesehen sein, die durch die Rohrsegmente geführt wird und längenmäßig so bestimmt ist, dass ein Lösen der Verschraubung aufgrund der damit einhergehenden Verlängerung des Deckenrohrs blockiert ist. Die Zugkordel kann beispielsweise endseitig an dem Deckenrohr und/oder an dem deckenmontierten Träger der Deckenbefestigungsvorrichtung und/oder einem der Befestigung des Medizingeräts dienenden Tragarmsystem festgelegt sein, welches an einem freien Ende des Deckenrohrs drehbar oder fest gehalten ist. Beispielsweise kann ein Stift oder eine Schraube als Verschraubungssicherungselement dienen und zwei benachbarte Rohrsegmente form- und/oder kraftschlüssig relativ zueinander festlegen.

Nach einer bevorzugten Ausführungsform der Erfindung ist das Gewinde als ein Feingewinde ausgebildet. Vorteilhaft wirkt bereits die Ausgestaltung des Gewindes als Feingewinde dem unbeabsichtigten Lösen der Verschraubung aufgrund der erhöhten Reibung im Gewinde entgegen.

Nach einer Weiterbildung der Erfindung ist das Verschraubungssicherungselement als ein innenliegendes Verschraubungssicherungselement realisiert. Im Sinne der Erfindung ist ein Verschraubungssicherungselement innenliegend, wenn es in das Deckenrohr beziehungsweise die das Deckenrohr bildenden Rohrsegmente so integriert ist, dass es für einen außenstehenden Betrachter nicht sichtbar angeordnet ist. Vorteilhaft wird durch die innenliegende Realisierung des Verschraubungssicherungselements eine hochwertige und ansprechende Optik des Deckenrohrs gewährleistet. Mithin kann das segmentierte Deckenrohr auf den ersten Blick nicht von einem klassischen, auf Länge gefertigten Deckenrohr unterschieden werden. Darüber hinaus ist einer Ansammlung von Schmutz oder anderen Schadstoffen im Bereich des Verschraubungssicherungselements bei der innenliegenden Anordnung entgegengewirkt. Dies ist insbesondere bei den üblichen Einsatzorten, insbesondere in einem Operationssaal, in einer Notaufnahme oder einem anderen medizinischen Behandlungsraum von Vorteil.

Nach einer Weiterbildung der Erfindung ist an den Rohrsegmenten eine Zentrierfläche vorgesehen, die eingerichtet ist, benachbarte Rohrsegmente konzentrisch zueinander anzuordnen. Die Zentrierfläche ist bevorzugt in Umfangsrichtung der Rohrsegmente orientiert und beispielsweise umlaufend gestaltet. Die Zentrierfläche kann mantelseitig beispielsweise von einem stirnseitig an dem Rohrsegment vorgesehenen Wellenabsatz bereitgestellt werden.

Nach der Erfindung sind an einander gegenüberliegenden Rohrsegmentstirnseiten zueinander parallele und senkrecht zur einer Längsmittelachse des Deckenrohrs orientierte Anlageflächen ausgebildet. Die Anlageflächen dienen insbesondere dazu, die zu verschraubenden Rohrsegmente zueinander fluchtend beziehungsweise ohne Winkelversatz auszurichten.

Nach der Erfindung sieht jedes Rohrsegment ein Innengewinde und ein Außengewinde vor. Das Außengewinde ist dabei beispielsweise einer ersten Rohrsegmentstirnseite und das Innengewinde einer der ersten Rohrsegmentstirnseite gegenüberliegenden zweiten Rohrsegmentstirnseite zugeordnet. Vorteilhaft kann durch die stets gleiche Gestalt der verschiedenen Rohrsegmente eine beliebige Kombinierbarkeit der Rohrsegmente ohne Adapterstücke oder dergleichen gewährleistet werden. Hierdurch vereinfacht sich die Montage des Deckenrohrs aus den einzelnen Rohrsegmenten.

Nach einer Weiterbildung der Erfindung steht das Verschraubungssicherungselement mit dem Gewinde im Wirkeingriff. Beispielsweise kann das Gewinde ein insbesondere metallisches Klemmteil aufweisen, welches beim Herstellen der Verschraubung einen Reibschluss herstellt und einem Lösen der Verschraubung auf diese Weise entgegenwirkt.

Nach einer Weiterbildung der Erfindung kann ein Kunststoffring und insbesondere ein Polyamidring als Verschraubungssicherungselement und Klemmring vorgesehen sein. Der Kunststoffring kann beispielsweise in das Innengewinde oder benachbart hierzu an dem Rohrsegment vorgesehen sein. Bei der Montage benachbarter Rohrsegmente gelangt der Kunststoffring dann in einen Wirkeingriff mit dem Außengewinde des benachbarten Rohrsegments und stellt die Verschraubungssicherung über den Reibschluss her.

Nach einer Weiterbildung der Erfindung ist ein Klebstoff als stoffschlüssig wirkendes Verschraubungssicherungselement vorgesehen. Der Klebstoff kann beispielsweise auf das Außengewinde des Rohrsegments aufgebracht werden. Die Aufbringung des Klebstoffs kann werkseitig erfolgen. In diesem Fall kann der Klebstoff mit einer an der Baustelle zu entfernenden Schutzfolie gegen ein Austrocknen, eine Verschmutzung und ein unbeabsichtigtes Anhaften beim Transport gesichert werden. Beispielsweise kann der Klebstoff im Rahmen der Montage auf der Baustelle vor Ort aufgebracht werden.

Es kann vorgesehen sein, dass zwei oder mehr Verschraubungssicherungselemente vorgesehen werden. Die zwei oder mehr Verschraubungssicherungselemente können gleichartig ausgebildet sein oder von unterschiedlicher Art sein. Beispielsweise können eine Verklebung mit einem Klemmring kombiniert werden.

Bevorzugt haben die Rohrsegmente, die zu dem Deckenrohr verschraubt werden, einen gleichen Außendurchmesser.

Zur Lösung der Aufgabe weist die Erfindung die Merkmale des Patentanspruchs 10 auf. Demzufolge umfasst eine erfindungsgemäße Installationsanordnung eine deckenmontierte Deckenbefestigungsvorrichtung, wobei der Träger der Deckenbefestigungsvorrichtung an einer Decke befestigt ist und ein Deckenrohr, welches an dem Träger gehalten ist, sich vertikal längs erstreckt. Das Deckenrohr weist erfindungsgemäß die Mehrzahl von Rohrsegmenten auf. Die Rohrsegmente sind miteinander verschraubt. Benachbarte Rohrsegmente sehen ein Verschraubungssicherungselement vor, das einem unbeabsichtigten Lösen der Verschraubung entgegenwirkt.

Die erfindungsgemäße Deckenbefestigungsvorrichtung wird insbesondere zur deckenmontierten Aufhängung von Medizingeräten, beispielsweise in Operationssälen, Notaufnahmen oder anderen medizinischen Behandlungsräumen, vorgesehen. Darüber hinaus kann eine Verwendung der Deckenbefestigungsvorrichtung in der Industrie und im Handwerk vorgesehen. Beispielsweise kann ein Diagnoseterminal in einer Kfz-Werkstatt oder ein Bediengerät eines Fertigungsanlage mittels der erfindungsgemäße Deckenbefestigungsvorrichtung aufgehangen werden.

Aus den weiteren Unteransprüchen und der nachfolgenden Beschreibung sind weitere Vorteile, Merkmale und Einzelheiten der Erfindung zu entnehmen. Die dort erwähnten Merkmale können jedenfalls einzeln für sich oder auch in beliebiger Kombination erfindungswesentlich sein. Erfindungsgemäß beschriebene Merkmale und Details der Deckenbefestigungsvorrichtung gelten selbstverständlich auch im Zusammenhang mit der Installationsanordnung und umgekehrt. So kann auf die Offenbarung zu den einzelnen Erfindungsaspekten stets wechselseitig Bezug genommen werden. Die Zeichnungen dienen lediglich beispielhaft der Klarstellung der Erfindung und haben keinen einschränkenden Charakter.

Es zeigen:

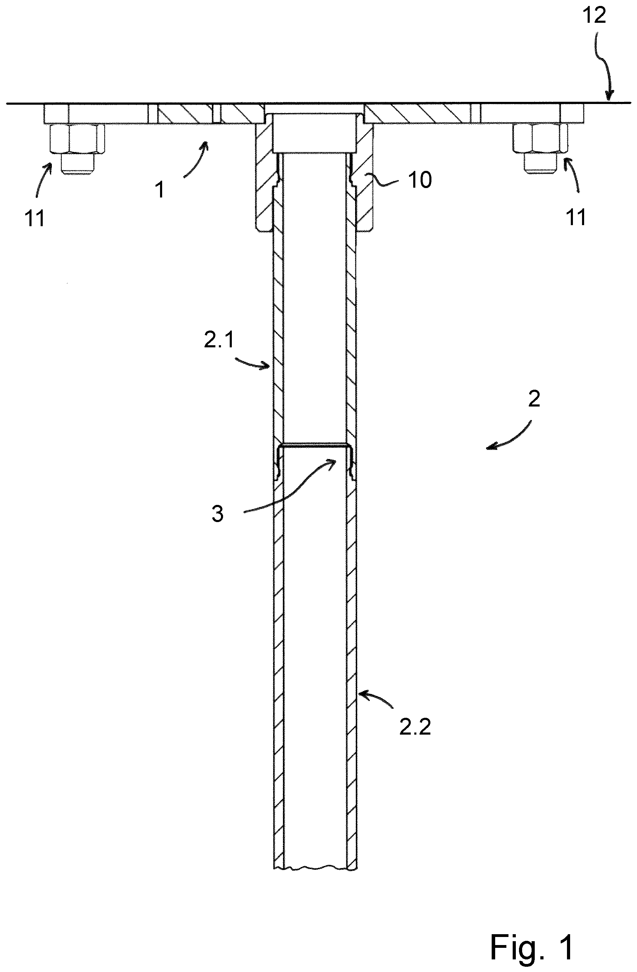

- Fig. 1: eine Schnittdarstellung einer Deckenbefestigungsvorrichtung mit einem deckenmontierten Träger und einem vertikal orientierten, segmentierten Deckenrohr,

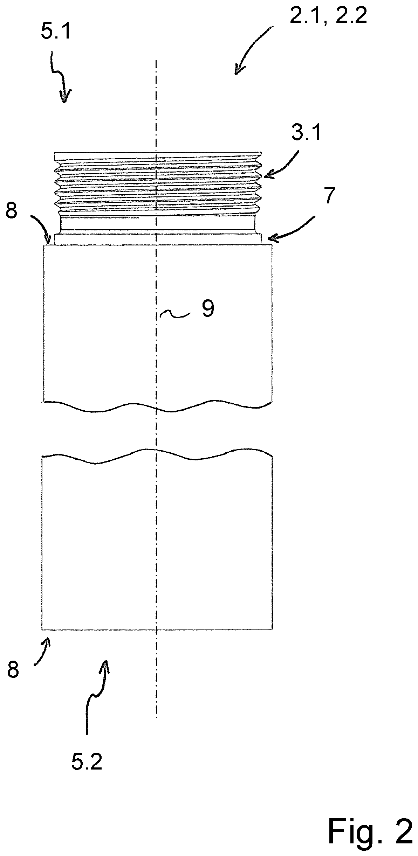

- Fig. 2: ein Rohrsegment des Deckenrohrs der Deckenbefestigungsvorrichtung nach Fig. 1 und

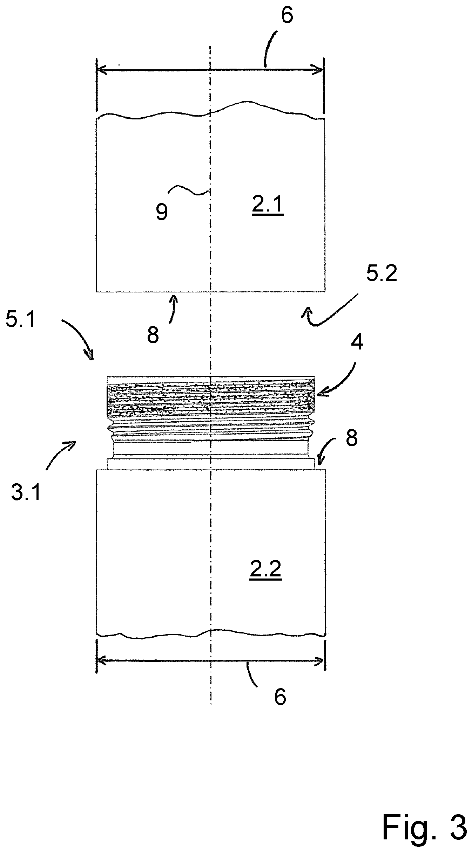

- Fig. 3: eine Teildarstellung zweier Rohrsegmente des Deckenrohrs mit einem Verschraubungssicherungselement in einer Vormontagesituation.

Eine erfindungsgemäße Deckenbefestigungsvorrichtung sowie eine Installationsanordnung hiermit umfassen als wesentliche Funktionskomponenten einen Träger 1, der über eine Deckenverschraubung 11 an einer Decke 12 gehalten ist, sowie ein im montierten Zustand vertikal längserstreckt orientiertes Deckenrohr 2. Das Deckenrohr 2 ist über eine Rohraufnahme 10 an dem Träger 1 festgelegt.

Das Deckenrohr 2 ist als segmentiertes Deckenrohr 2 realisiert. Es umfasst eine Mehrzahl von Rohrsegmenten 2.1, 2.2, die über ein Gewinde 3 miteinander verschraubt sind. Jedes Rohrsegment 2.1, 2.2 sieht hierbei an gegenüberliegenden Rohrsegmentstirnseiten 5.1, 5.2 ein Außengewinde 3.1 sowie ein in den Zeichnungen nicht dargestelltes Innengewinde vor, welches korrespondierend zu dem Außengewinde 3.1 gestaltet ist und der Verbindung benachbarter Rohrsegmente 2.1, 2.2 dient. Das Gewinde 3 ist bevorzugt als Feingewinde realisiert.

Die einzelnen Rohrsegmente 2.1, 2.2 können eine gleiche oder unterschiedliche Länge aufweisen. Darüber hinaus ist ein Außendurchmesser 6 der Rohrsegmente 2.1, 2.2 bevorzugt gleich groß, sodass die Rohrsegmente 2.1, 2.2 im montierten Zustand ein Deckenrohr 2 mit einem konstanten Außendurchmesser 6 bilden.

An den Rohrsegmenten 2.1, 2.2 ist zu Montagezwecken eine in Umfangsrichtung umlaufend gestaltete, in Richtung der Längsmittelachse 9 flächig erstreckte Zentrierfläche 7 sowie eine senkrecht hierzu und zur Längsmittelachse 9 orientierte stirnseitige Anlagefläche 8 gebildet. Die Zentrierfläche 7 und die Anlagefläche 8, die zueinander senkrecht orientiert sind, dienen der konzentrischen und planparallelen Anlage benachbarter Rohrsegmente 2.1, 2.2 zueinander. Zur Vereinfachung der Montage kann vorgesehen sein, dass beispielsweise an der dem Innengewinde zugeordneten Zentrierfläche 7 eine Montagefase angeordnet ist, die eine Selbstausrichtung der Rohrsegmente 2.1, 2.2 zueinander beim Verschrauben derselben herstellt.

Um die zwei Rohrsegmente 2.1, 2.2 miteinander zu verschrauben und sie gegen ein unbeabsichtigtes Lösen der Verschraubung zu sichern, kann ein Klebstoff 4 als ein Verschraubungssicherungselement der Deckenbefestigungsvorrichtung vorgesehen werden. Der Klebstoff 4 wird vor der Montage beispielsweise auf das Außengewinde 3.1 aufgebracht.

Die Aufbringung des Klebstoffs kann werkseitig erfolgen. In diesem Fall ist es vorteilhaft, den Klebstoff 4 mit einer Schutzfolie zu überdecken. Die Schutzfolie verhindert ein Austrocknen des Klebstoffs 4 und ein Anhaften von Fremdkörpern an dem Klebstoff 4. Sie wird unmittelbar vor der Montage entfernt. Alternativ kann der Klebstoff 4 als Verschraubungssicherungselement auf der Baustelle unmittelbar vor dem Montieren der Rohrsegmente 2.1, 2.2 auf das Außengewinde 3.1 aufgebracht werden.

Die Verwendung des Klebstoffs 4 als stoffschlüssig wirkendes Verschraubungssicherungselement ist lediglich exemplarisch zu verstehen. Alternativ können andere Verschraubungssicherungselemente verwendet werden, die einem unbeabsichtigten Lösen der Verschraubung der benachbarten Rohrsegmente 2.1, 2.2 entgegenwirken. Beispielsweise kann nach Art einer Sicherungsmutter ein Kunststoffbeziehungsweise Polyamidring als Verschraubungssicherungselement verwendet werden. Beispielsweise kann ein auf das Gewinde 3 wirkendes metallisches Klemmteil als Verschraubungssicherungselement verwendet werden.

Die Rohrsegmente 2.1, 2.2, die zu dem Deckenrohr 2 verschraubt werden, können bevorzugt eine unterschiedliche Länge aufweisen. Hierdurch ist es möglich, die gewünschte absolute Länge des Deckenrohrs durch die Verbindung unterschiedlich langer Rohrsegmente 2.1, 2.2 miteinander variabel und bedarfsgerecht einzustellen. Die Auswahl der geeigneten Rohrsegmente 2.1, 2.2 kann bei der Montage der Deckenbefestigungsvorrichtung vor Ort auf der Baustelle erfolgen. Es ist insofern nicht notwendig, ein Aufmaß zu nehmen beziehungsweise die Länge des Deckenrohrs im Vorfeld zu bestimmen, ein für den speziellen Verwendungsfall individuell auf Länge gefertigtes Deckenrohr herzustellen oder ein überlang bereitgestelltes Deckenrohr vor Ort auf der Baustelle auf Maß zu schneiden.

Gleiche Bauteile und Bauteilfunktionen sind durch gleiche Bezugszeichen gekennzeichnet.

## Patentansprüche

1. Deckenbefestigungsvorrichtung für Medizingeräte umfassend einen deckenmontierbaren Träger (1) sowie ein in einer Montageposition vertikal längserstrecktes und an dem Träger (1) gehaltenes Deckenrohr (2), wobei das Deckenrohr (2) eine Mehrzahl von Rohrsegmenten (2.1, 2.2) aufweist, wobei die Rohrsegmente über ein Gewinde (3) miteinander verschraubt sind und benachbarte Rohrsegmente (2.1, 2.2) ein Verschraubungssicherungselement vorsehen, das eingerichtet ist, einem Lösen der Verschraubung entgegenzuwirken, wobei jedes Rohrsegment (2.1, 2.2) ein Innengewinde und ein Außengewinde (3.1) vorsieht und wobei das Außengewinde (3.1) einer ersten Rohrsegmentstirnseite (5.1) und das Innengewinde einer der ersten Rohrsegmentstirnseite gegenüberliegenden zweiten Rohrsegmentstirnseite (5.2) zugeordnet ist, **dadurch gekennzeichnet, dass** die Rohrsegmentstirnseiten (5.1, 5.2) zueinander parallele und senkrecht zu einer Längsmittelachse (9) des Deckenrohrs (2) orientierte Anlageflächen (8) aufweisen und dass das Deckenrohr (2) wenigstens zwei Rohrsegmente (2.1, 2.2) mit einer unterschiedlichen Länge vorsieht.

2. Deckenbefestigungsvorrichtung nach Anspruch 1, **dadurch gekennzeichnet, dass** das Gewinde (3) als ein Feingewinde ausgebildet ist.

3. Deckenbefestigungsvorrichtung nach Anspruch 1 oder 2, **dadurch gekennzeichnet, dass** das Verschraubungssicherungselement mit dem Gewinde (3) im Wirkeingriff steht.

4. Deckenbefestigungsvorrichtung nach einem der Ansprüche 1 bis 3, **dadurch gekennzeichnet, dass** ein Klebstoff (4) als Verschraubungssicherungselement vorgesehen ist.

5. Deckenbefestigungsvorrichtung nach Anspruch 4, **dadurch gekennzeichnet, dass** der Klebstoff (4) auf das Außengewinde (3.1) und/oder das Innengewinde aufgetragen ist.

6. Deckenbefestigungsvorrichtung nach einem der Ansprüche 3 bis 5, **dadurch gekennzeichnet, dass** ein Kunststoffring und insbesondere ein Polyamidring als Verschraubungssicherungselement vorgesehen ist.

7. Deckenbefestigungsvorrichtung nach einem der Ansprüche 1 bis 6, **dadurch gekennzeichnet, dass** das Verschraubungssicherungselement innenliegend realisiert ist.

8. Deckenbefestigungsvorrichtung nach einem der Ansprüche 1 bis 7, **dadurch gekennzeichnet, dass** an den Rohrsegmenten (2.1, 2.2) eine Zentrierfläche (7) vorgesehen ist, wobei die Zentrierfläche (7) eingerichtet ist, benachbarte Rohrsegmente (2.1, 2.2) konzentrisch zueinander anzuordnen.

9. Deckenbefestigungsvorrichtung nach einem der Ansprüche 1 bis 8, **dadurch gekennzeichnet, dass** benachbarte Rohrsegmente (2.1, 2.2) und bevorzugt alle Rohrsegmente (2.1, 2.2) einen gleichen und/oder konstanten Außendurchmesser (6) aufweisen.

10. Installationsanordnung umfassend eine deckenmontierte Deckenbefestigungsvorrichtung nach einem der Ansprüche 1 bis 9.

11. Verwendung einer Deckenbefestigungsvorrichtung nach einem der Ansprüche 1 bis 9 zur deckenmontierten Aufhängung von Medizingeräten.

## Claims

1. A ceiling fastening apparatus for medical devices, comprising a ceiling-mountable carrier (1) and a ceiling tube (2) extending in a vertical longitudinal direction in an assembly position and held on the carrier (1), the ceiling tube (2) having a plurality of tube segments (2.1, 2.2), the tube segments being screwed together via a thread (3) and adjacent tube segments (2.1, 2.2) providing for a screw connection securing element to be configured to counteract a loosening of the screw connection, each tube segment (2.1, 2.2) providing for an internal thread and an external thread (3.1) and the external thread (3.1) being assigned to a first tube segment end face (5.1) and the internal thread being assigned to a second tube segment end face (5.2) opposite the first tube segment end face, **characterized in that** the tube segment end faces (5.1, 5.2) have contact surfaces (8) parallel to each other and oriented perpendicularly to a longitudinal center axis (9) of the ceiling tube (2) and **in that** the ceiling tube (2) provides for at least two tube segments (2.1, 2.2) having a different length.

2. The ceiling fastening apparatus according to claim 1, **characterized in that** the thread (3) is formed as a fine-pitch thread.

3. The ceiling fastening apparatus according to claim 1 or 2, **characterized in that** the screw connection securing element is in operative engagement with the thread (3).

4. The ceiling fastening apparatus according to any of claims 1 to 3, **characterized in that** an adhesive (4) is provided for as a screw connection securing element.

5. The ceiling fastening apparatus according to claim 4, **characterized in that** the adhesive (4) is applied to the external thread (3.1) and/or the internal thread.

6. The ceiling fastening apparatus according to any of claims 3 to 5, **characterized in that** a plastic ring and in particular a polyamide ring is provided for as a screw connection securing element.

7. The ceiling fastening apparatus according to any of claims 1 to 6, **characterized in that** the screw connection securing element is implemented internally.

8. The ceiling fastening apparatus according to any of claims 1 to 7, **characterized in that** a centering surface (7) is provided for on the tube segments (2.1, 2.2), the centering surface (7) being configured to arrange adjacent tube segments (2.1, 2.2) concentrically to each other.

9. The ceiling fastening apparatus according to any of claims 1 to 8, **characterized in that** adjacent tube segments (2.1, 2.2) and preferably all the tube segments (2.1, 2.2) have a same and/or constant outer diameter (6).

10. An installation arrangement comprising a ceiling-mounted ceiling fastening apparatus according to any of claims 1 to 9.

11. A use of a ceiling fastening apparatus according to any of claims 1 to 9 for ceiling-mounted suspension of medical devices.

## Revendications

1. Dispositif de fixation au plafond pour appareils médicaux comprenant un support (1) pouvant être monté au plafond ainsi qu'un tube de plafond (2) s'étendant verticalement en une position de montage et fixé au support (1), le tube de plafond (2) présentant une pluralité de segments tubulaires (2.1, 2.2), les segments tubulaires étant vissés l'un à l'autre par un filetage (3) et un élément de sécurisation du vissage étant prévu aux segments tubulaires (2.1, 2.2) voisins pour empêcher un dévissage, chaque segment tubulaire (2.1, 2.2) présentant un taraudage et un filetage (3.1), le filetage (3.1) à l'extrémité (5.1) d'un premier segment tubulaire correspondant au taraudage à l'extrémité (5.2) d'un second segment tubulaire, **caractérisé en ce que** les extrémités (5.1, 5.2) des segments tubulaires présentant des surfaces de contact (8) parallèles entre elles et perpendiculaires à un axe longitudinal médian (9) du tube de plafond (2) et le tube de plafond (2) présentant au moins deux segments tubulaires (2.1, 2.2) de longueur différente.

2. Dispositif de fixation au plafond selon la revendication 1, **caractérisé en ce que** le filetage (3) est réalisé sous forme de filetage fin.

3. Dispositif de fixation au plafond selon la revendication 1 ou 2, **caractérisé en ce que** l'élément de sécurisation du vissage est en prise fonctionnelle avec le filetage (3).

4. Dispositif de fixation au plafond selon l'une des revendications 1 à 3, **caractérisé en ce qu'**une colle (4) est prévue comme élément de sécurisation du vissage.

5. Dispositif de fixation au plafond selon la revendication 4, **caractérisé en ce que** la colle (4) est appliquée sur le filetage (3.1) et/ou sur le taraudage.

6. Dispositif de fixation au plafond selon l'une des revendications 3 à 5, **caractérisé en ce qu'**une bague en matière plastique et notamment en polyamide est prévue comme élément de sécurisation du vissage.

7. Dispositif de fixation au plafond selon l'une des revendications 1 à 6, **caractérisé en ce que** l'élément de sécurisation du vissage est réalisé sous forme d'élément intérieur.

8. Dispositif de fixation au plafond selon l'une des revendications 1 à 7, **caractérisé en ce que** les segments tubulaires (2.1, 2.2) sont dotés d'une surface de centrage (7), la surface de centrage (7) étant conçue pour agencer concentriquement l'un par rapport à l'autre les segments tubulaires (2.1, 2.2) voisins.

9. Dispositif de fixation au plafond selon l'une des revendications 1 à 8, **caractérisé en ce que** les segments tubulaires (2.1, 2.2) voisins et de préférence tous les segments tubulaires (2.1, 2.2) présentent un diamètre extérieur (6) identique et/ou constant.

10. Configuration comprenant, monté au plafond, un dispositif de fixation au plafond selon l'une des revendications 1 à 9.

11. Utilisation d'un dispositif de fixation au plafond selon l'une des revendications 1 à 9 pour la suspension au plafond d'appareils médicaux.
